# EUROPEAN PATENT APPLICATION

(11) **EP 4 667 908 A1**
(43) Date of publication of application: **24.12.2025**
(21) Application number: 24183817.6
(22) Date of filing: 21.06.2024
(51) Int. Cl.: G01N 21/31, G01N 21/85, B07C 5/342, G01N 33/02, G01N 21/359

(54) **METHODS AND SYSTEMS FOR DETECTING SOLIDS IN FOOD PRODUCTS BASED ON HYPERSPECTRAL IMAGES**

(71) Applicant: NULAB ANALITICA ALIMENTARIA, S.L.U., 31110 Noain (ES)
(72) Inventor: BENGOECHEA ÁLVAREZ, María José, 31110 Noain (ES); UCERO ZAMARBIDE, Mikel, 31110 NOAIN (ES)
(74) Representative: Elion IP, S.L.

(57) **Abstract**

The disclosure refers to methods and systems for detecting solids in food products based on hyperspectral images. The method comprises providing a product stream comprising the food products, illuminating the product stream with a light source emitting incident light; generating hyperspectral images of each one of the food products of the product stream from the reflected incident light from the food products; and selecting wavelength ranges of interest from the spectral patterns of the food products, the wavelength ranges being indicative of the presence of the solids in the food products. Then, for each food product under test of the food stream, the method comprises comparing the wavelength ranges of interest from the spectral pattern of the food product under test with the same wavelength ranges of interest from the same food product free of solids; and determining the presence of a solid based on the result of the comparison.

## Description

### TECHNICAL FIELD

The present invention refers generally to the field of computer vision for food inspection and, more particularly, to a method and a system for detecting solids in food products based on hyperspectral images.

### BACKGROUND

Physical foreign material contamination is a persistent problem that affects all types of food processing industries. Foreign material is defined as non-food, foreign bodies that may cause illness or injury to the consumer, and materials that are not typically part of the food product. Detection of this physical foreign materials is especially complex when they are located inside the food product or when these physical foreign materials have low densities, i.e., densities lower than 1g/m³, and/or they are of small size, i.e., smaller than 5 mm in any of their dimensions. For example, in the agri-food sector, fruits with stones that have been previously removed by mechanical means may have small pieces or chips of stone inside that are very difficult to detect. Certain vegetables, such as lettuce or leeks, may have small stones, soil or invertebrates between their leaves that are also very difficult to detect.

Numerous methods exist to prevent foreign objects from contaminating foods or to detect them once they enter the food processing line. Most detection methods are tailored to individual applications and are only suitable to be used in certain food products and will be ineffective with others.

X-ray imaging technology has been used in the food and agriculture sectors during the last decades for food inspection. In particular, this X-ray imaging technology has been used for the detection and identification of generic foreign materials or contaminants, mainly of inorganic origin, which are generally mixed with the product during the treatment and production processes. Generally speaking, these generic foreign materials may be mineral rocks, crystals, rubbers, and metallic objects that may be mixed with the product. There exist a wide range of X-ray inspection systems for food from a plurality of manufacturers in the market. X-ray inspection systems work by sending X-rays through products to create X-ray images. The resulting images are inspected manually or automatically, and then compared to predetermined standards of acceptance. As the X-ray beam passes through a product, the X-ray energy gets absorbed, and the remaining energy that reaches the system array generates a grayscale image. The light and dark variations within this X-ray image indicate whether or not the product contains foreign material, missing components, product or packaging defects, or the proper fill volume.

However, the X-ray imaging technology has some drawbacks which are the risk of radiation exposure for operators and its negative impact on the environment. Besides, this technology is expensive, uses high voltages, is not effective in identifying low-density materials (with densities lower than 1 g/m³), especially when these materials are organic materials (e.g., hairs, fruit stone chips, insects, etc.) which are mixed with the product, and presents some problems for detecting wood chips (e.g., fruit stone chips) or plastic chips.

Other alternative technologies have been used to try to overcome some of the drawbacks of the X-ray imaging technology. One of these solutions has been the use of Artificial Vision. Artificial Vision is the technology that allows computers to make decisions from small amount of visual data. It works by capturing images through cameras and sensors, which are then processed with sophisticated algorithms to analyze and understand them. However, RGB cameras are not able to discriminate physical foreign materials when they have the same color than the product in which they are located (e.g., fruit stone chips, weeds, insects, etc.).

Other existing technologies for food inspection are based on spectroscopic techniques. These techniques consist in emitting beams of light incident on the product or sample under test and then, capturing the spectrum of the reflected light. By analyzing said spectrum (spectral fingerprint) some properties of the sample or product under test can be obtained. However, these spectroscopic techniques are mainly focused on capturing spectra of individual elements or products or portions thereof in laboratory settings instead of scanning complete products or product streams in industrial environments. Besides, these existing solutions are not able to combine spectroscopy techniques with vision techniques which make these techniques unable to detect small size, i.e., foreign materials whose size in any of their dimensions is smaller than 5 mm. This problem is especially critical in the agri-food industry since these spectroscopy-based techniques cannot detect remains of fruit stones that are left inside the fruit after it has been removed by mechanical means, or remains of soil, mineral stones or insects inside vegetables, such as lettuce.

Given the mentioned limitations of the existing technologies for detecting low density and small foreign materials in food products, these are generally manually inspected by operators in the industrial processing line of such food products. This solution is inefficient, time consuming, expensive and error prone.

Therefore, there is a need in the art for methods and systems for food inspection which that are capable of detecting foreign materials in food, in particular low-density materials (lower than lower than 1g/m³) and of small size (smaller than 5 mm in any of their dimensions), that do not affect the quality of the food products being inspected and that are safe for the health of operators and environmentally friendly.

### DESCRIPTION

A first aspect of the present disclosure refers to a method for detecting solids in food products based on hyperspectral images. As used herein, the solids may refer to any solid foreign material which is a non-food body and that may cause illness or injury to the consumer and are not typically part of the food. These solids may be part of the raw materials such as fruit pits, seeds, bones, or parts or chips thereof, or may be contaminants like stones, soil, grit, insects, or pieces of the food harvesting or processing equipment like metal or plastic chips, among others.

As used herein, hyperspectral images refer to images obtained by a hyperspectral camera that is technique that collects and processes information across the electromagnetic spectrum to obtain the spectrum for each pixel in an image. This technique allows for the identification of objects and materials by analysing their unique spectral signatures.

The method comprises providing a product stream comprising the food products. The food products may be, for example, fruits and/or vegetables. Preferably, the fruits may be stone fruits such as peaches, nectarines, apricots, plums, cherries, olives, etc, from which the stone has been previously removed by mechanical means. Thus, the solids to be detected may be, preferably, fruit stone chips located inside the fruits or soil, stones or invertebrates located inside vegetables such as lettuce or leeks, among others.

Then, the method illuminates the product stream with a light source emitting incident light. Hyperspectral technology operates in the visible and near-infrared portion (VNIR) of the electromagnetic spectrum having wavelengths between 400 and 1700 nm (NIR), that includes visible spectrum wavelength band (400-700 nm approximately) and part of the near-infrared wavelength band (900-1700 nm approximately). Although the light emitting source may emit white light, preferably, it will emit light having wavelengths between 100 and 900 nm.

By using at least one hyperspectral camera, hyperspectral images of each one of the food products of the product stream are generated with the incident light reflected from the food products. The hyperspectral images provide spectral patterns of the food products. Then, at least one wavelength range of interest from the spectral patterns of the food products is selected. The selected at least one wavelength range of interest correspond to at least one portion of the spectral patterns being indicative of the presence of the solids in the food products. The selected wavelength range of interests will depend on the type of product under analysis, the type of solid to be detected in the product and the specific application of the method herein disclosed.

Then, for each food product under test of the food stream, the method further comprises:
detecting a maximum reflectance intensity within the at least one wavelength range of interest from the spectral pattern obtained from the generated hyperspectral images of the food product under test;
comparing the maximum reflectance intensity with a first pre-established range; and
determining the presence of a solid in the food product under test when the maximum reflectance intensity is within the first pre-established range. Therefore, when the maximum reflectance intensity associated to the food product under test is within the first pre-established range, it can be stated that there is a solid in the food product. The definition of this first pre-established range may be made by previously comparing the spectral patterns obtained from hyperspectral images of food products with solids with the spectral patterns obtained from hyperspectral images of food products free of solids. In this way, it can be easily determined the reflectance intensity range that corresponds to the presence of solids in the food products. Additionally, the definition of this first pre-stablished range may be determined based on multiple variables that at least comprise: the type of product under analysis, the environmental conditions, the specific setup of the cameras (including camera configuration, distance to the product, exposure time, number of frames per second), the incident angle of the light, the product flow rate, the type of solid to be detected in the product and the specific application of the method herein disclosed, among many others.

This method allows detecting the presence or absence of these solids in a reliable and easy way. It further allows detecting the presence of these solids inside the food products and independently of the particular shapes, densities, and geometries of the food products. Besides, due to the nature of the hyperspectral imaging technology, solids with sizes lower than 5 mm in any of their dimensions and with densities lower than 1g/m³ can be detected. Those food products in which these solids are detected may be discarded or post-processed to remove the detected solids.

In some embodiments, when more than one wavelength range of interest have been selected, in particular three or more than three wavelength ranges of interest, the method further comprises:
generating at least three hyperspectral images associated to the corresponding at least three corresponding wavelength ranges of interest. These hyperspectral images, which are generated from one single spectral band, correspond to one channel of the hyperspectral camera;
combining three of the hyperspectral images (three channels of the hyperspectral camera) to obtain a RGB image. This transformation of the three hyperspectral images into the RGB image is performed so that the spectral image is transformed in a representation of the visible spectrum so it can be observed by the human eye. When there are more than three wavelength ranges of interest, the method may perform multiple combinations of hyperspectral images to obtain different RGB images that could be used individually or collectively to determine the presence of the solids;
determining the presence of the solid by comparing the colour of the RGB image with a second pre-established range.

This second pre-established range, that is a wavelength range, can be obtained by comparing the colours associated to the spectral patterns obtained from hyperspectral images of food products with solids with the spectral patterns obtained from the generated hyperspectral images of food products free of solids. For example, it could be determined that the purple colour corresponds to the presence of the solids, which is equivalent to say that if the wavelength band corresponding to the RGB image falls within the range 380-465 nm, then the presence of the solid can be determined. Alternatively, a different colour (with its corresponding spectral band) may be associated to the presence or absence of the solids.

In some embodiments, selecting the at least one wavelength range of interest comprises:
comparing the spectral patterns obtained from hyperspectral images of the food products free of solids and spectral patterns obtained from hyperspectral images obtained from the same food products having solids; and
selecting the at least one wavelength range of interest based on the result of the comparison.

In some embodiments, a particular wavelength range is determined to be of interest when an intensity difference between the spectral pattern of the food product free of solids and the spectral pattern of the same food product having solids in that particular wavelength of interest is greater than a third pre-established range. The third pre-stablished range, that may be a wavelength range, may be also determined based on multiple variables that at least comprise: the type of product under analysis, the environmental conditions, the specific setup of the cameras (including camera configuration, distance to the product, exposure time, number of frames per second), the incident angle of the light, the product flow rate, the type of solid to be detected in the product and the specific application of the method herein disclosed, among many others

In some embodiments, the method comprises generating, using the hyperspectral camera, hyperspectral images of the surroundings of the light source free of food products, the hyperspectral images providing a spectral pattern of the surroundings; and
prior to comparing the at least one wavelength range of interest of the spectral pattern corresponding to the food product under test with the at least one range of interest of the spectral pattern corresponding to the same food product free of solids, the method comprises removing pixels (in the spatial domain) corresponding to the spectral pattern of the surroundings of the light source free of food products from the spectral pattern corresponding to the product under test and from the spectral pattern of the product free of solids which are under the threshold. By eliminating these spatial pixels bands in the spectral patterns of the food product under test and of the food product free of solids, only relevant data related to the product-solid is considered in the comparison stage. For example, data related to the belt transporting the products, data produced by reflections in the image or data generated by other defects is discarded.

In some embodiments, removing spatial pixels from the spectral patterns which are under the threshold comprises:
generating a digital mask from the hyperspectral image of the surroundings of the light source free of food products, wherein those spatial pixels of the hyperspectral image of the surroundings of the light source free of food products which are below the threshold are zero-values in the digital mask and those spatial pixels of the hyperspectral image of the surroundings of the light source free of food products which are above the threshold are non-zero values in the digital mask. As used herein, a digital mask is a binary image consisting of zero-values and non-zero values. If a mask is applied to another image of the same size, all pixels which are zero in the mask are set to zero in the output image. All others remain unchanged; and
combining the hyperspectral images corresponding to the food product under test and to the same food product free of solids with the generated digital mask.

In some embodiments, the detected solids are solids having a density below 1 kg/m³.

In some embodiments, the solids have a size lower than 5 mm, preferably between 1 and 4 mm.

In some embodiments, the solids can be organic or inorganic solids selected from a group comprising bone fruits chips, metallic chips, vitreous chips, mineral rocks, invertebrates and combinations thereof.

A second aspect of the disclosure is a system for detecting solids in food products based on hyperspectral images. The system comprises a light source that is configured to illuminate a product stream comprising the food products to be inspected with incident light of a single or different wavelengths. The light emitting source will, preferably, emit light having wavelengths between 400 and 1700nm.

The system further comprises a hyperspectral camera configured to generate hyperspectral images of each one of the food products of the product stream from the reflected incident light from the product products. The hyperspectral imaging camera can be a whiskbroom camera, a pushbroom camera, a hyperspectral camera based on spectral scanning or a snapshot camera. Preferably, the hyperspectral imaging camera is a hyperspectral camera based on spectral scanning.

The system additionally comprises a controller configured to:
select at least one wavelength range of interest from an spectra pattern of the hyperspectral images, the at least one wavelength range of interest corresponding to the portion of the spectra pattern generated by the presence of the solids to be detected in the food product;
detect, for each food product of the product stream, a maximum reflectance intensity within the at least one wavelength range of interest from the spectral pattern obtained from the generated hyperspectral images of the food product under test;
compare the maximum reflectance intensity with a first pre-established range; and
determine the presence of a solid in the food product under test when the maximum reflectance intensity is within the first pre-established range.

The controller may include hardware and software logic to perform the functionalities described above in relation the method for detecting solids in food products based on hyperspectral images.

In some embodiments, the hyperspectral images are generated using near infrared spectroscopy. The spectral range of a hyperspectral imaging system or camera refers to the range of wavelengths of light that it can capture. This range can vary depending on the specific system and application, but typically covers the visible to near-infrared spectrum (400-2500 nm).

In some embodiments, the light source may be a halogen light source or a LED light source. While the halogen light sources are capable of emitting light in the entire NIR spectrum and have a longer useful life, the LED light sources can be designed to emit specific wavelengths, are more efficient in term of electrical consumption, generate less heat, allow to control and modulate its intensity and have a longer useful life. However, current LED light sources do not emit in all the NIR spectra. Preferably, the light source will be a halogen light source.

A third aspect of the disclosure is a data processing system comprising means for carrying out the method for detecting solids in food products based on hyperspectral images previously disclosed.

The solution herein disclosed present some advantages over other existing solutions. In particular, it is able to detect solids of organic or inorganic origin with densities lower than 1g/m³ and with sizes lower than 5 mm in any of their dimensions. Besides, the solution herein disclosed allows inspecting food products in industrial process in a quick, accurate and reliable manner and that is able to work with large quantities of food products to be inspected (e.g., it is able to operate with processing speed greater than 12 m/min).

### BRIEF DESCRIPTION OF THE DRAWINGS

To complete the description and in order to provide for a better understanding of the disclosure, a set of drawings is provided. Said drawings form an integral part of the description and illustrate an example of the disclosure, which should not be interpreted as restricting the scope of the disclosure, but just as an example of how the disclosure can be carried out.

The drawings comprise the following figures:
Figure 1 shows a flow diagram of a method for detecting solids in food products based on hyperspectral images, according to an embodiment of the disclosure.
Figure 2 shows a block diagram of a system for detecting solids in food products based on hyperspectral images, according to an embodiment of the disclosure
Figure 3 shows a machine for processing a food product that incorporates a system for detecting solids in food products based on hyperspectral images, according to an embodiment of the disclosure.
Figure 4 shows a detailed view of the set of hyperspectral cameras of the system for detecting solids in food products based on hyperspectral images of Figure 3.
Figure 5 shows a graph including the normalized spectral pattern of a peach and the normalized spectral pattern of the stone of a peach.

### DESCRIPTION OF EXAMPLES

In the following description, for purposes of explanation, numerous specific details are set forth in order to provide a thorough understanding of the present systems and methods. It will be apparent, however, to one skilled in the art that the present systems, and methods may be practiced without these specific details. Reference in the specification to "an example", "an embodiment" or similar language means that a particular feature, structure, or characteristic described in connection with that embodiment is included as described but may not be included in other embodiments.

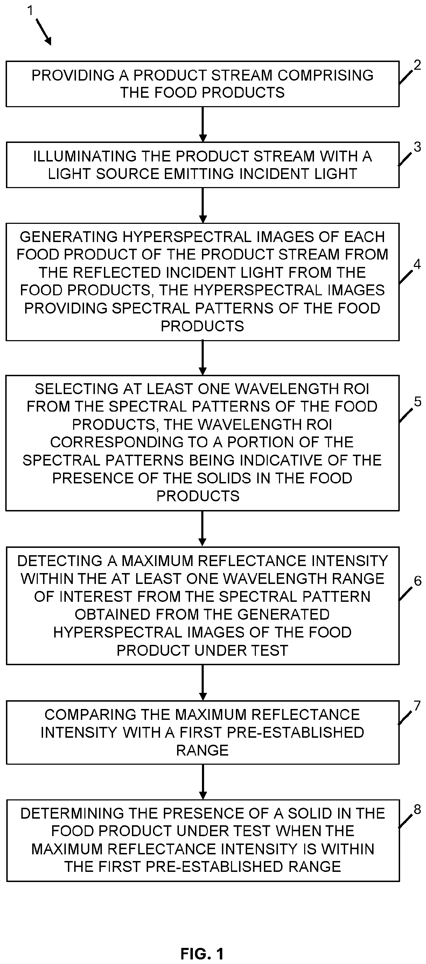

Figure 1 shows a flow diagram of a method 1 for detecting solids in food products based on hyperspectral images.

At step 2 of the method 1, a product stream comprising the food products is provided. For example, the food products may be peaches, so a continuous stream of peaches is provided. These peaches may be located on a continuously moving belt, so that the peaches pass underneath the hyperspectral cameras, at a distance from the cameras within their operating range. The food product may be any other edible product that may contain physical foreign materials, including vegetables or stone fruits but also meat, fish, bakery products, milk products, etc. For the particular case of the peaches, the solids to be detected may be stone fruit chips located inside the peaches or soil, stones or invertebrates located or inside or outside the peaches. The solids may be also plastic or metal chips coming from the industrial treatment process to which they are subjected.

At step 3 of the method 1, the product stream is illuminated with a light source emitting incident light. Hyperspectral technology operates in the visible and near-infrared portion (VNIR) of the electromagnetic spectrum having wavelengths between 400 and 1700 nm, that includes visible spectrum wavelength band (400-700 nm approximately) and part of the near-infrared wavelength band (900-1700 nm approximately). Although the light emitting source may emit white light, preferably, it will emit light having wavelengths between 400 and 1000 nm. While in some examples the hyperspectral cameras may incorporate or be coupled to these light sources, in some other examples, the hyperspectral cameras and the light sources may be independent elements. In any of these cases, the light sources and the hyperspectral cameras will be adequately oriented over the food product stream to adequately direct the light beam on the individual food products.

At step 4 of the method 1, the hyperspectral cameras generate hyperspectral images of each one the peaches of the stream passing beneath them by capturing the incident light that is reflected from the food products. The hyperspectral images provide spectral patterns of the food products.

At step 5 of the method 1, one or more wavelength Range of Interest (ROI) from the spectral patterns of the peaches are selected. The selected wavelength ROls correspond to the portions of the spectral patterns that are indicative of the presence of the solids, e.g., fruit bone chips, insects, stones, etc., inside the peaches. To do so, previously, the spectral patterns of peaches having different types of solids have been compared with spectral patterns of the peaches free of solids. Those portions of the spectra patterns that present some reflectance intensity differences (the differences will have to be greater than a predefined threshold) will be the selected ROls. The ROIs referring to different types of solids may differ from each other.

Then, for each food product under test of the food stream, the method further comprises the following steps 6 to 8.

At step 6 of the method 1, a maximum reflectance intensity within the previously selected wavelength ROI(s), corresponding to the food product under test, is detected.

At step 7 of the method 1, this maximum reflectance intensity is compared with a first pre-established range. This first pre-established range, which is a reflectance intensity range, will be previously obtained by comparing spectral patterns of peaches free of solids with spectral patterns of the peaches having solids. By making these comparisons, it can be easily determined which are the reflectance intensity ranges in that particular ROIs that corresponds to the presence of the solids in the peaches.

At step 8 of the method 1, the presence of the solid in the peach under test is determined when the maximum reflectance intensity is within the first pre-established range.

This method allows detecting the presence of these solids (insects, stones, stone fruit chips, weeds, etc.) inside the peaches and independently of the particular shapes, densities, and geometries of the peaches. It is important to notice that due to the nature of the hyperspectral imaging technology, solids with sizes lower than 5 mm in any of their dimensions and with densities lower than 1g/m³ can be detected by the method 1 herein disclosed. The peaches in which these solids are detected may be discarded or post-processed to remove the detected solids from them.

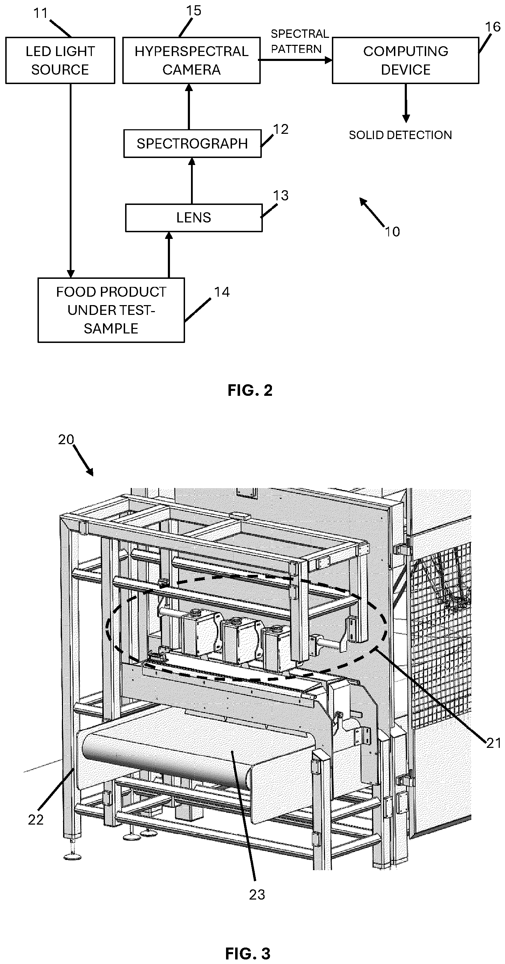

Figure 2 shows a block diagram of a system 10 for detecting solids in food products based on hyperspectral images, according to an embodiment of the disclosure. It should be understood that the system 10 may include additional components and that some of the components described herein may be removed and/or modified without departing from a scope of the system 10. Additionally, implementation of the example system 10 is not limited to such example as shown in Figure 2.

The system 10 comprises a halogen light source 11 that is configured to generate incident light on the food product under test (sample food product) 14. The reflected incident light is collimated by a lens 13 that directs said reflected incident light towards a spectrograph 12, that splits light into its component wavelengths, and to the hyperspectral imaging camera 15. This hyperspectral imaging camera is, preferably, a hyperspectral camera based on spectral scanning. The hyperspectral imaging camera 15 comprises the software to generate the hyperspectral images and the spectral patterns associated to the food product under test 14 from the hyperspectral images.

Additionally, the system 10 comprises a computing device 16 having a controller (not shown). The computing device 16 receives the spectral pattern of the food product under test 14 and carries out the method 1 disclosed in Figure 1.

Figure 3 shows a machine 20 for processing a food product that incorporates a system for detecting solids in food products based on hyperspectral images, according to an embodiment of the disclosure. It should be understood that the machine 20 may include additional components and that some of the components described herein may be removed and/or modified without departing from a scope of the system 20. Additionally, implementation of the example system 20 is not limited to such example as shown in Figure 3.

This machine comprises a frame 22, a belt 23 on which the fruit stream (not shown in this figure) is deposited and the system 21 for detecting solids in food products based on hyperspectral images. As depicted in Figure 3, the three hyperspectral cameras 24a-c are mounted on a structure attached to the frame 22 and oriented towards the belt 22.

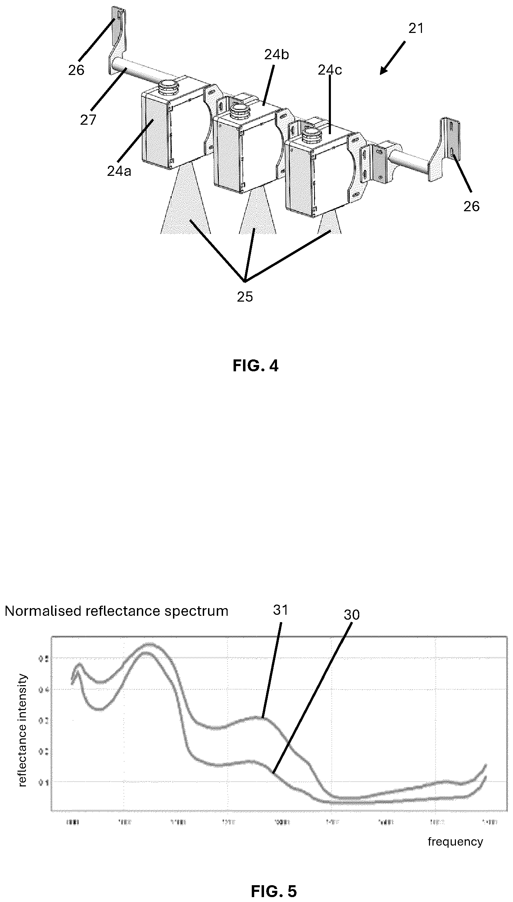

Figure 4 shows a detailed view of the set of hyperspectral cameras 24a-c of the system 21 for detecting solids in food products based on hyperspectral images of Figure 3. The three hyperspectral cameras 24a-c are integrated into respective casings that also incorporate the respective lenses, spectrographs and the halogen light sources. The halogen light sources emit an incident light beam 25 over the food products located onto the belt 22. These three hyperspectral cameras 24a-c are mounted on a horizontal axis 25 that is coupled, e.g., screwed or welded, to the frame 22 with interposition of respective fastening elements 26.

Figure 5 shows the normalized spectral pattern corresponding to a peach 30 and to the stone of the peach 31.

In this text, the term "comprises" and its derivations (such as "comprising", etc.) should not be understood in an excluding sense, that is, these terms should not be interpreted as excluding the possibility that what is described and defined may include further elements.

The invention is obviously not limited to the specific embodiments described herein, but also encompasses any variations that may be considered by any person skilled in the art within the general scope of the invention as defined in the claims.

## Claims

1. A method for detecting solids in food products based on hyperspectral images, **characterized in that** the method comprises the following steps:
providing a product stream comprising the food products;
illuminating the product stream with a light source emitting incident light;
generating, using a hyperspectral camera, hyperspectral images of each one of the food products of the product stream from the reflected incident light from the food products, the hyperspectral images providing spectral patterns of the food products;
selecting at least one wavelength range of interest from the spectral patterns of the food products, the at least one wavelength range of interest corresponding to at least one portion of the spectral patterns being indicative of the presence of the solids in the food products;
wherein for each food product under test of the food stream, the method further comprises:
detecting a maximum reflectance intensity within the at least one wavelength range of interest from the spectral pattern obtained from the generated hyperspectral images of the food product under test;
comparing the maximum reflectance intensity with a first pre-established range; and
determining the presence of a solid in the food product under test when the maximum reflectance intensity is within the first pre-established range.

2. The method according to claim 1, wherein when there are three or more than three wavelength range of interest, the method further comprises:
generating a hyperspectral image associated to at least three of the wavelength range of interest;
combining three hyperspectral images to obtain a RGB image;
determining the presence of the solid by comparing the colour of the RGB image with a second pre-established range.

3. The method according to any one of the preceding claims, wherein selecting the at least one wavelength range of interest comprises:
comparing the spectral patterns obtained from hyperspectral images of the food products free of solids with spectral patterns obtained from hyperspectral images of the same food products having solids; and
selecting the at least one wavelength range of interest based on the result of the comparison.

4. The method according to claim 3, comprising determining that a particular wavelength range is of interest when a reflectance intensity difference between the spectral pattern of the food product free of solids with the spectral pattern of the same food product having solids in that particular wavelength range is within a third pre-established range.

5. The method according to any one of the preceding claims, comprising:
generating, using the hyperspectral camera, hyperspectral images of the surroundings of the light source free of food products, the hyperspectral images providing a spectral pattern of the surroundings;
comparing the spectral patterns obtained from hyperspectral images of the food products having solids with the spectral patterns obtained from hyperspectral images of the surroundings of the light source free of food products;
detecting a wavelength bandwidth presenting a greater reflectance intensity difference between the two spectral patterns,
determining a threshold based on the reflectance intensity difference;
prior to comparing the at least one wavelength range of interest of the spectral pattern corresponding to the food product under test with the at least one range of interest of the spectral pattern corresponding to the same food product free of solids, the method comprises removing spatial pixels bands from the spectral pattern corresponding to the product under test which are under the threshold.

6. The method according to claim 5, wherein removing spatial pixels from the spectral pattern corresponding to the product under test which are under the threshold comprises:
generating a digital mask from the hyperspectral image of the surroundings of the light source free of food products, wherein those spatial pixels of hyperspectral image of the surroundings of the light source free of food products which are below the threshold are zero-values in the digital mask and those spatial pixels of hyperspectral image of the surroundings of the light source free of food products which are above the threshold are non-zero values in the digital mask; and
combining the hyperspectral image of the food product under test and of the food product free of solids with the generated digital mask.

7. The method according to any one of the preceding claims, wherein the detected solids are solids having a density below 1 kg/m³.

8. The method according to any one of the preceding claims, wherein the solids have a size lower than 5 mm, preferably between 1 and 4 mm, in any of the dimensions of the solids.

9. The method according to any one of the preceding claims, wherein the solids are selected from a group comprising bone fruits chips, metallic chips, vitreous chips, mineral rocks, rubber chips, invertebrates and combinations thereof.

10. The method according to any one of the preceding claims, wherein the food product is a fruit or a vegetable.

11. A system for detecting solids in food products based on hyperspectral images, **characterized in that** it comprises:
a light source configured to illuminate a product stream comprising the food products with incident light;
a hyperspectral camera configured to generate hyperspectral images of each one of the food products of the product stream from the reflected incident light from the product products; and
a controller configured to:
select at least one wavelength range of interest from the spectral patterns of the food products, the at least one wavelength range of interest corresponding to at least one portion of the spectral patterns being indicative of the presence of the solids in the food products;
detect, for each food product of the product stream, a maximum reflectance intensity within the at least one wavelength range of interest from the spectral pattern obtained from the generated hyperspectral images of the food product under test;
compare the maximum reflectance intensity with a first pre-established range; and
determine the presence of a solid in the food product under test when the maximum reflectance intensity is within the first pre-established range.

12. The system according to claim 11, wherein the hyperspectral images are generated using near infrared spectroscopy.

13. The system according to claim 11 or 12, wherein the light source is a halogen light source or a LED light source, preferably a halogen light source.

14. A data processing system comprising means for carrying out the method according to any one of claims 1 to 10.
